# EUROPEAN PATENT APPLICATION

(11) **EP 3 273 675 A1**
(43) Date of publication of application: **24.01.2018**
(21) Application number: 17176153.9
(22) Date of filing: 14.06.2017
(51) Int. Cl.: H04N 5/32, H04N 5/345

(54) **RADIATION DETECTOR AND RADIOGRAPHIC IMAGING DEVICE**

(30) Priority: 19.07.2016 JP 2016141319
(71) Applicant: Toshiba Electron Tubes & Devices Co., Ltd., Otawara-shi, Tochigi 324-8550 (JP)
(72) Inventor: Fujita, Shuichi, Tochigi (JP)
(74) Representative: Henkel, Breuer & Partner

(57) **Abstract**

According to one embodiment, a radiation detector includes a detector including a plurality of pixels and converting radiation into electrical information, a read circuit reading the information for each of the plurality of pixels, an image memory storing a plurality of the read information, and
a thinning circuit limiting an output of the plurality of information stored in the image memory.

## Description

### FIELD

An embodiment of the invention relates to a radiation detector and a radiographic imaging device.

### BACKGROUND

An array substrate that includes many pixels, a drive control circuit that applies control signals to the pixels, a read circuit that reads image data signals from the pixels, etc., are provided in a radiation detector.

Here, several thousand×several thousand pixels are provided in the array substrate. Because it is standard to have a bit count of sixteen bits, quite a large amount of information is transmitted. Therefore, it takes time to transmit the information; and much time is necessary from the imaging to the displaying as an image. In such a case, the transmission time can be shortened by using high-speed communication using an optical fiber as the communication part. However, it is problematic that costs increase for the high-speed communication using the optical fiber compared to generally popularized methods such as Ethernet (registered trademark), etc. Also, if wireless communication is used, it is difficult to multiply provide the radiation detectors because the communication channel is undesirably occupied for a long period of time; and there is a risk that the communication speeds of other devices may decrease.

Therefore, technology has been proposed in which image data signals from predetermined multiple pixels are omitted; image data signals are collected from predetermined multiple pixels; or the number of pixels for which the image data signals are read is reduced.

However, the resolution of the radiation image is reduced thereby. Also, in such technology, the image data from the unselected pixels is deleted. Therefore, in the case where the radiation image that has the original resolution of the radiation detector becomes necessary, it is necessary to re-perform the radiographic imaging.

Therefore, it is desirable to develop technology to obtain a radiation image of the necessary quality for which the transmission time of the information can be short.

### BRIEF DESCRIPTION OF THE DRAWINGS

FIG. 1 is a block diagram for illustrating an X-ray imaging device according to the embodiment;
FIG. 2 is a schematic perspective view for illustrating the X-ray detector according to the embodiment;
FIG. 3 is a block diagram of the X-ray detector;
FIG. 4 is a circuit diagram of an array substrate;
FIG. 5 is a block diagram for illustrating the image storage/transmission circuit;
FIG. 6 is a block diagram for illustrating the image storage/transmission circuit according to another embodiment;
FIG. 7 is a schematic view for illustrating the memory addresses of the information stored in the image memory;
FIG. 8A to FIG. 11D are X-ray images and schematic views for illustrating the information thinned by the image storage/transmission circuit;
FIG. 12 is a schematic view for illustrating the gradation of the information stored in the image memory; and
FIG. 13A to FIG. 16D are X-ray images and schematic views for illustrating the information thinned by the image storage/transmission circuit.

### DETAILED DESCRIPTION

According to one embodiment, a radiation detector includes a detector including a plurality of pixels and converting radiation into electrical information, a read circuit reading the information for each of the plurality of pixels, an image memory storing a plurality of the read information, and
a thinning circuit limiting an output of the plurality of information stored in the image memory.

Embodiments will now be illustrated with reference to the drawings. Similar components in the drawings are marked with the same reference numerals; and a detailed description is omitted as appropriate.

A radiation detector according to the embodiment is applicable to various radiation such as γ-rays, etc., as well as X-rays. Here, as an example, a case relating to X-rays is described as a typical example of radiation. Accordingly, applications to other radiation also are possible by replacing "X-ray" of the embodiments recited below with "other radiation."

An X-ray detector 1 illustrated below is an X-ray planar sensor that detects an X-ray image which is a radiation image. X-ray planar sensors can be largely divided into a direct conversion method and an indirect conversion method.

The direct conversion method is a method in which the photoconductive charge (the signal charge) generated in the photoconductive film interior by incident X-rays is directly guided by a high electric field to a storage capacitor for charge storage.

The indirect conversion method is a method in which the X-rays are converted into fluorescence (visible light) by a scintillator; the fluorescence is converted into a signal charge by a photoelectric conversion element such as a photodiode, etc.; and the signal charge is guided to a storage capacitor.

Although the indirect conversion X-ray detector 1 is illustrated as an example in the description recited below, the invention is applicable also to direct conversion X-ray detectors.

In other words, it is sufficient for the X-ray detector to include multiple pixels and to include a detector that converts the radiation into electrical information.

Known technology is applicable to the direct conversion X-ray detector; and a detailed description is therefore omitted.

Although the X-ray detector 1 that is used in a general medical application or the like is illustrated as an example in the description recited below, the applications of the X-ray detector 1 are not limited.

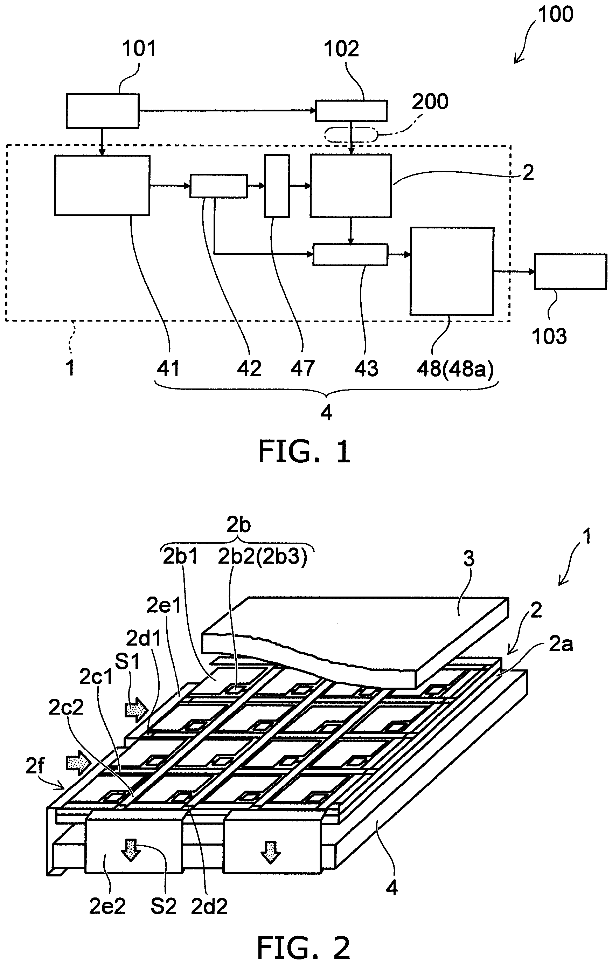

FIG. 1 is a block diagram for illustrating an X-ray imaging device 100 according to the embodiment.

As shown in FIG. 1, a controller 101, an X-ray generator 102, a display part 103, and the X-ray detector 1 are provided in the X-ray imaging device 100.

The controller 101 is electrically connected to the high-voltage power supply of the X-ray generator 102 and an internal signal generation circuit 41 of the X-ray detector 1. The controller 101 may be, for example, a computer including a CPU (Central Processing Unit) and a memory device, etc.

The controller 101 inputs a synchronization signal to the high-voltage power supply and the internal signal generation circuit 41. The operation of the X-ray generator 102 and the operation of the X-ray detector 1 are synchronized by the input synchronization signal. Therefore, the X-ray detector 1 executes the operations necessary for the imaging synchronously with the incidence of the X-rays irradiated from the X-ray generator 102.

The X-ray generator 102 generates the X-rays. The X-ray generator 102 may include, for example, an X-ray source and a high-voltage power supply. The X-ray source may be, for example, a vacuum tube that generates X-rays. The X-ray source includes a filament and a target. The filament is formed from, for example, tungsten, etc., and is electrically connected to the negative side of the high-voltage power supply. The target is formed from, for example, copper, tungsten, molybdenum, etc., and is electrically connected to the positive side of the high-voltage power supply. The electrical power (the tube current or the tube voltage) that is necessary for the irradiation of the X-rays is supplied from the high-voltage power supply to the X-ray source. The X-ray source generates electrons in the filament and causes the electrons accelerated by the supplied high voltage to collide with the target to irradiate X-rays toward a screening subject 200 inside the effective field-of-view region. A not-illustrated collimator that adjusts the shape of the X-ray beam irradiated from the X-ray source may be provided between the X-ray source and the X-ray detector 1.

The display part 103 is electrically connected to the X-ray detector 1 (a transmission output circuit 51). The display part 103 generates an X-ray image which is an optical image based on the signal from the X-ray detector 1 (the transmission output circuit 51) and displays the X-ray image that is generated.

As described below, image storage/transmission circuits 48 and 48a output, in stages by dividing into multiple outputs, multiple information stored in an image memory 49. However, generally, the display part 103 includes a correction circuit. Therefore, the correction circuit can generate the X-ray image based on the signal output from the X-ray detector 1 in stages by dividing into multiple outputs. Also, the correction circuit can increase, in stages, at least one of the resolution of the X-ray image or the gradation of the X-ray image based on the signal output from the X-ray detector 1 in stages by dividing into multiple outputs.

Known technology can be used as the correction circuit; and a detailed description is therefore omitted.

The display part 103 may be, for example, a flat panel display, etc. The details relating to the generation of the X-ray image are described below.

The X-ray detector 1 detects the X-rays irradiated from the X-ray generator 102 and transmitted by the screening subject 200.

FIG. 2 is a schematic perspective view for illustrating the X-ray detector 1 according to the embodiment.

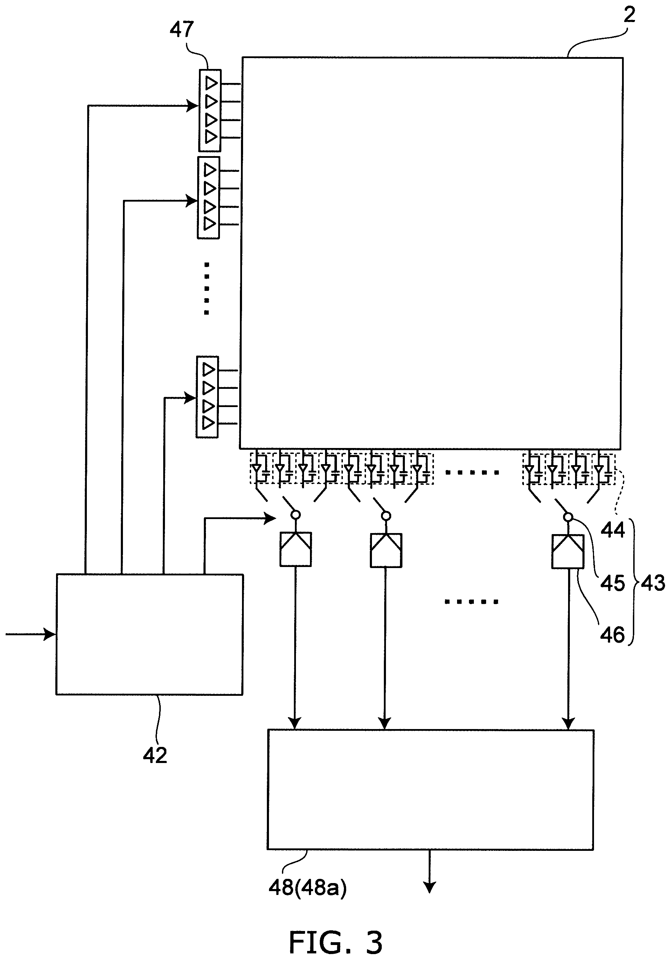

FIG. 3 is a block diagram of the X-ray detector 1.

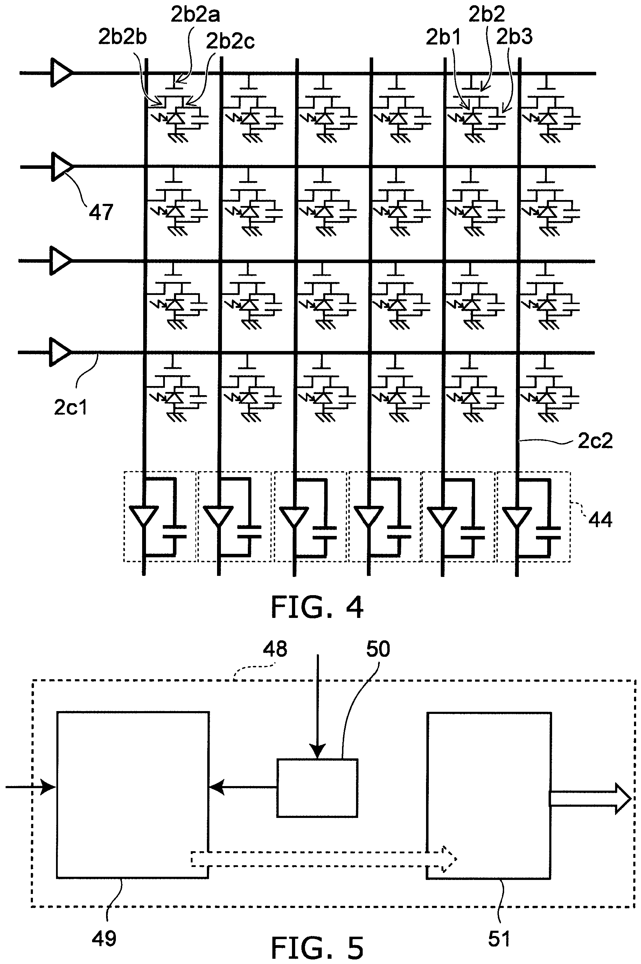

FIG. 4 is a circuit diagram of an array substrate 2.

As shown in FIG. 2 to FIG. 4, the array substrate 2, a scintillator 3, and a circuit board 4 are provided in the X-ray detector 1.

The array substrate 2 is provided at a surface on the side where the X-rays are incident of a not-illustrated support plate fixed in the interior of a not-illustrated housing. The circuit board 4 is provided at the surface on the side of the support plate opposite to the side where the array substrate 2 is provided.

The array substrate 2 converts, into an electrical signal, the fluorescence (the visible light) converted from the X-rays by the scintillator 3.

Because the X-ray detector 1 is an indirect conversion X-ray detector, the scintillator 3 and pixels 2b provided in the array substrate 2 are a "detector that includes multiple pixels and converts the radiation into electrical information."

The array substrate 2 includes a substrate 2a, pixels 2b, control lines (or gate lines) 2c1, and data lines (or signal lines) 2c2.

The substrate 2a has a plate configuration and is formed from a transparent material such as alkali-free glass, etc.

The pixels 2b are multiply provided on one surface of the substrate 2a.

The pixels 2b have rectangular configurations and are provided in regions defined by the multiple control lines 2c1 and the data lines 2c2. The multiple pixels 2b are arranged in a matrix configuration.

A photoelectric conversion element 2b1 and a thin film transistor (a TFT (Thin Film Transistor)) 2b2 which is a switching element are provided in each of the multiple pixels 2b.

As shown in FIG. 4, a storage capacitor 2b3 that stores the signal charge converted by the photoelectric conversion element 2b1 also may be provided. For example, the storage capacitor 2b3 has a rectangular flat plate configuration and may be provided under each of the thin film transistors 2b2. However, according to the capacitance of the photoelectric conversion element 2b1, the photoelectric conversion element 2b1 also may be used as the storage capacitor 2b3.

The photoelectric conversion element 2b1 may be, for example, a photodiode, etc.

The thin film transistor 2b2 performs the switching between the storing of the charge in the storage capacitor 2b3 and the discharging of the charge stored in the storage capacitor 2b3. The thin film transistor 2b2 includes a gate electrode 2b2a, a source electrode 2b2b, and a drain electrode 2b2c. The gate electrode 2b2a of the thin film transistor 2b2 is electrically connected to the corresponding control line 2c1. The source electrode 2b2b of the thin film transistor 2b2 is electrically connected to the corresponding data line 2c2. The drain electrode 2b2c of the thin film transistor 2b2 is electrically connected to the storage capacitor 2b3 and the corresponding photoelectric conversion element 2b1.

The control lines 2c1 are multiply provided to be parallel to each other at a prescribed spacing. For example, the control lines 2c1 extend in a row direction.

One control line 2c1 is electrically connected to one of multiple interconnect pads 2d1 provided at the peripheral edge vicinity of the substrate 2a. One of multiple interconnects provided in a flexible printed circuit board 2e1 is electrically connected to one interconnect pad 2d1. The other ends of the multiple interconnects provided in the flexible printed circuit board 2e1 are electrically connected respectively to gate drivers 47 provided in the circuit board 4.

The data lines 2c2 are multiply provided to be parallel to each other at a prescribed spacing. For example, the data lines 2c2 extend in a column direction orthogonal to the row direction.

One data line 2c2 is electrically connected to one of multiple interconnect pads 2d2 provided at the peripheral edge vicinity of the substrate 2a. One of multiple interconnects provided in a flexible printed circuit board 2e2 is electrically connected to one interconnect pad 2d2. The other ends of the interconnects provided in the flexible printed circuit board 2e2 are electrically connected to a read circuit 43 provided in the circuit board 4.

The control line 2c1 and the data line 2c2 may be formed using, for example, a low-resistance metal such as aluminum, chrome, etc.

A protective layer 2f covers the pixels 2b, the control lines 2c1, and the data lines 2c2. The protective layer 2f is formed from an insulating material.

The scintillator 3 is provided on the multiple photoelectric conversion elements 2b1 and converts the incident X-rays into fluorescence, i.e., visible light. The scintillator 3 is provided to cover the region (the effective pixel region) on the substrate 2a where the multiple pixels 2b are provided.

The scintillator 3 may be formed using, for example, cesium iodide (CsI):thallium (Tl), sodium iodide (NaI):thallium (Tl), etc. In such a case, the scintillator 3 that is made of an aggregate of multiple columnar crystals is formed if the scintillator 3 is formed using vacuum vapor deposition, etc.

The scintillator 3 also may be formed using, for example, gadolinium oxysulfide (Gd₂O₂S), etc. In such a case, a trench portion that has a matrix configuration may be formed so that the scintillator 3 having a quadrilateral prism configuration is provided for each of the multiple pixels 2b.

Also, to increase the utilization efficiency of the fluorescence and improve the sensitivity characteristics, a not-illustrated reflective layer may be provided to cover the front surface side (the incident surface side of the X-rays) of the scintillator 3.

A not-illustrated moisture-resistant body that covers the scintillator 3 and the not-illustrated reflective layer may be provided to suppress the degradation of the characteristics of the scintillator 3 and the characteristics of the not-illustrated reflective layer due to the water vapor included in the air.

The internal signal generation circuit 41, a drive control circuit 42, the read circuit 43, the gate drivers 47, and the image storage/transmission circuit 48 are provided in the circuit board 4.

The input side of the internal signal generation circuit 41 is electrically connected to the controller 101. The output side of the internal signal generation circuit 41 is electrically connected to the drive control circuit 42.

The internal signal generation circuit 41 controls the drive control circuit 42 based on a synchronization signal from the controller 101.

The input side of the drive control circuit 42 is electrically connected to the internal signal generation circuit 41. The output side of the drive control circuit 42 is electrically connected to the read circuit 43 and the gate drivers 47.

The drive control circuit 42 generates a timing signal that reads the charge stored in the pixels 2b based on the signal from the internal signal generation circuit 41.

In other words, the drive control circuit 42 generates a control signal S1 for each of the multiple gate drivers 47 based on the signal from the internal signal generation circuit 41. The drive control circuit 42 sequentially inputs the generated control signal S1 to each of the gate drivers 47.

The drive control circuit 42 also controls selection circuits 45 based on the signal from the internal signal generation circuit 41. The selection circuits 45 sequentially read image data signals S2 based on the signals from the drive control circuit 42.

The read circuit 43 reads, for each of the multiple pixels 2b (the multiple photoelectric conversion elements 2b1), the information converted into the electrical information by the multiple pixels 2b.

The input side of the read circuit 43 is electrically connected to the drive control circuit 42 and the data lines 2c2. The output side of the read circuit 43 is electrically connected to the image storage/transmission circuit 48.

Multiple integrating amplifiers 44, the multiple selection circuits 45, and multiple AD converters 46 are provided in the read circuit 43.

One integrating amplifier 44 is electrically connected to one data line 2c2. One selection circuit 45 is provided for the prescribed number of integrating amplifiers 44. One AD converter 46 is electrically connected to one selection circuit 45.

The integrating amplifiers 44 sequentially receive the image data signals S2 from the pixels 2b. The integrating amplifier 44 integrates the current flowing within a constant amount of time, and outputs a voltage corresponding to the integral to the selection circuit 45. Thus, it is possible to convert the value (the amount of charge) of the current flowing through the data line 2c2 within a prescribed amount of time into a voltage value. In other words, the integrating amplifier 44 converts, to potential information, the image data information corresponding to the intensity distribution of the fluorescence generated by the scintillator 3.

The selection circuit 45 selects the data line 2c2 for performing the reading, and sequentially reads the image data signal S2 converted into the potential information.

The AD converter 46 sequentially converts the image data signals S2 that are read into digital signals. The image data signals S2 that are converted into the digital signals are input to the image storage/transmission circuit 48.

The input sides of the gate drivers 47 are electrically connected to the drive control circuit 42. The output sides of the gate drivers 47 are electrically connected to the control lines 2c1.

The gate drivers 47 are multiply provided. One gate driver 47 is electrically connected to one control line 2c1. The gate driver 47 switches between the ON state and the OFF state of the thin film transistor 2b2. The gate driver 47 inputs the control signal S1 to the corresponding control line 2c1 according to the scanning direction of the X-ray image. By the control signal S1 that is input to the control line 2c1, the thin film transistor 2b2 is switched to the ON state; and the signal charge (the image data signal S2) from the pixel 2b can be output.

In other words, the internal signal generation circuit 41, the drive control circuit 42, and the gate drivers 47 select one row of the multiple pixels 2b arranged in the matrix configuration according to the synchronization signal from the controller 101. The read circuit 43 reads the information (the image data signal S2) from one pixel 2b selected by selecting one column of the multiple pixels 2b. Thus, the read circuit 43 sequentially reads the information from all of the pixels 2b for the selected row.

When the information from all of the pixels 2b for the selected row has been read, the internal signal generation circuit 41, the drive control circuit 42, and the gate drivers 47 select the next row of the multiple pixels 2b. The read circuit 43 sequentially reads the information from all of the pixels 2b for the selected row.

Thereafter, this operation is repeated; and the image read of one screen is completed by reading the information from the pixels 2b of all of the rows. The information from all of the pixels 2b provided in the array substrate 2 (the image data signal S2 of one screen) is input to the image storage/transmission circuit 48.

The input side of the image storage/transmission circuit 48 (48a) is electrically connected to the read circuit 43. The output side of the image storage/transmission circuit 48 (48a) is electrically connected to the display part 103.

FIG. 5 is a block diagram for illustrating the image storage/transmission circuit 48.

As shown in FIG. 5, the image memory 49, a thinning circuit 50, and the transmission output circuit 51 are provided in the image storage/transmission circuit 48.

The input side of the image memory 49 is electrically connected to the read circuit 43 (the multiple AD converters 46) and the thinning circuit 50. The output side of the image memory 49 is electrically connected to the input side of the transmission output circuit 51.

The image memory 49 stores the multiple information (the information from all of the pixels 2b provided in the array substrate 2) output from the read circuit 43. In other words, the multiple information stored in the image memory 49 is the image data signals S2 of one screen of the X-ray image.

The thinning circuit 50 limits the output of the multiple information stored in the image memory 49. Also, as described below, the thinning circuit 50 further outputs the information of the multiple information stored in the image memory 49 not output the previous time.

The thinning circuit 50 generates the signal for thinning the information stored in the image memory 49 and inputs the generated signal to the image memory 49. For example, the thinning circuit 50 generates signals designating the memory addresses of the multiple information for outputting a portion of the information stored in the image memory 49 and inputs the generated signals to the image memory 49. In other words, the thinning circuit 50 designates the memory addresses of the multiple information output from the image memory 49.

The image memory 49 inputs the information stored at the designated memory addresses to the transmission output circuit 51 based on the signals from the thinning circuit 50. In other words, the image memory 49 outputs the information stored at the designated memory addresses based on the signals designating the memory addresses.

The transmission output circuit 51 transmits the information output from the image memory 49 to the display part 103.

According to a predetermined method or a command from an operator, the image storage/transmission circuit 48 outputs, as supplemental information, the information stored in the image memory 49 at memory addresses that were not designated. The supplemental information is input to the display part 103. At this time, the information stored in the image memory 49 can be output in stages by dividing into multiple outputs.

In other words, the thinning circuit 50 limits the output of the multiple information according to the predetermined method or the command from the operator.

The thinning circuit 50 outputs the multiple information stored in the image memory 49 in stages by dividing into multiple outputs.

The thinning circuit 50 outputs the multiple information for each desired memory address in stages by dividing into multiple outputs.

Thus, if the image data signals S2 of one screen are transmitted to the outside by being thinned, the amount of the transmitted information can be small. Therefore, the transmission time of the information can be reduced.

The display part 103 generates an X-ray image which is an optical image based on the information output from the transmission output circuit 51 and displays the X-ray image that is generated. Generally, the X-ray image can be displayed even if the information is thinned because a correction circuit is provided in the display part 103. Therefore, it is possible to drastically shorten the time (the image display time) from the irradiation of the X-rays until the X-ray image is displayed.

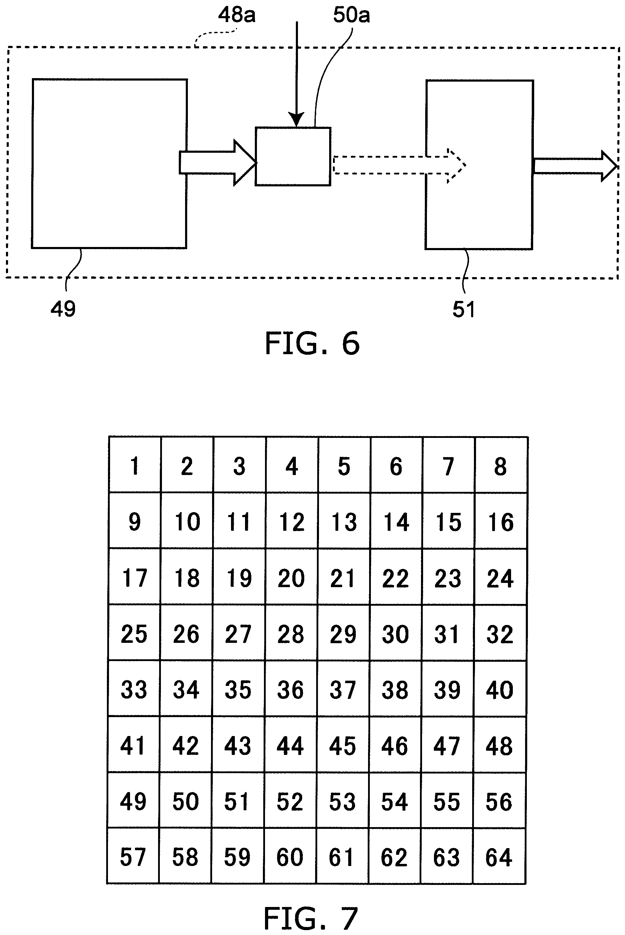

FIG. 6 is a block diagram for illustrating the image storage/transmission circuit 48a according to another embodiment.

As shown in FIG. 6, the image memory 49, a thinning circuit 50a, and the transmission output circuit 51 are provided in the image storage/transmission circuit 48a.

The input side of the image memory 49 is electrically connected to the read circuit 43 (the multiple AD converters 46). The output side of the image memory 49 is electrically connected to the input side of the thinning circuit 50a. The output side of the thinning circuit 50a is electrically connected to the input side of the transmission output circuit 51.

The image memory 49 stores the information (the information from all of the pixels 2b provided in the array substrate 2) output from the read circuit 43. In other words, the image memory 49 stores the image data signals S2 of one screen.

The thinning circuit 50a limits the output of the multiple information stored in the image memory 49. Also, as described below, the thinning circuit 50a further outputs the information of the multiple information stored in the image memory 49 not output the previous time.

The thinning circuit 50a designates the bit numbers of the multiple information output from the image memory 49 to input a portion of the information stored in the image memory 49 to the transmission output circuit 51. The information of the designated bit numbers is input to the transmission output circuit 51.

The transmission output circuit 51 transmits the information of the designated bit numbers to the display part 103.

The image storage/transmission circuit 48a outputs, as supplemental information, the information of the remaining bit numbers stored in the image memory 49 according to the predetermined method or the command from the operator. The supplemental information is input to the display part 103. At this time, the information stored in the image memory 49 can be output in stages by dividing into multiple outputs.

In other words, the thinning circuit 50a limits the output of the multiple information according to the predetermined method or the command from the operator.

The thinning circuit 50a outputs the multiple information stored in the image memory 49 in stages by dividing into multiple outputs.

The thinning circuit 50a outputs the multiple information for each desired bit number in stages by dividing into multiple outputs. For example, the thinning circuit 50a outputs, in stages, from the information of the high-order bit numbers.

According to the embodiment as well, effects similar to those of the image storage/transmission circuit 48 illustrated in FIG. 5 can be obtained.

Here, the resolution of the X-ray image generated based on the information thinned by the image storage/transmission circuit 48 is low. Also, the gradation of the X-ray image generated based on the information thinned by the image storage/transmission circuit 48a is low. Therefore, there is a risk that the small portions of the X-ray image may be difficult to confirm.

However, the information that is not used when generating the X-ray image of the previous time is stored in the image memory 49. Therefore, in the case where it is necessary to increase the resolution and/or gradation of the X-ray image, supplementing can be performed using the remaining information stored in the image memory 49. In other words, as necessary, it is possible to increase in stages the resolution and/or gradation of the X-ray image by additionally using the information not used when generating the X-ray image of the previous time. Therefore, without re-imaging, the resolution and/or the gradation can be increased in stages; and ultimately, the X-ray image that uses all of the image data signals S2 can be displayed.

The level of the thinning (the limit amount of the output of the multiple information) can be changed according to the confirmation content and/or the application of the X-ray image. Therefore, it is also possible to select the level of the thinning according to many types of purposes such as diagnosis time priority, resolution priority, gradation priority, etc.

For example, it is also possible to reduce the confirmation time by changing the limit amount of the output of the multiple information according to the confirmation content of the X-ray image.

In such a case, the thinning circuits 50 and 50a also can change the limit amount of the output of the multiple information based on at least one of the requested transmission time of the multiple information, the requested resolution of the X-ray image, or the requested gradation of the X-ray image.

In the case where fine portions of the X-ray image are problematic (the diagnosis of a tumor, etc.), the image storage/transmission circuit 48a in which the resolution is not changed may be used.

For example, in the case where the resolution of the X-ray image is given priority, the thinning circuit 50a designates the bit numbers of the multiple information output from the image memory 49.

In the case where the change of the color of the X-ray image is problematic (the diagnosis of a bone fracture, etc.), the image storage/transmission circuit 48 in which the shading of the color does not change may be used.

For example, in the case where the gradation of the X-ray image is given priority, the thinning circuit 50 designates the memory addresses of the multiple information output from the image memory 49.

Multiple thinning methods may be combined. For example, an image storage/transmission circuit in which the image memory 49, the thinning circuit 50, the thinning circuit 50a, and the transmission output circuit 51 are provided may be used.

Also, according to the application of the X-ray image, etc., one of the thinning circuit 50 or the thinning circuit 50a may be used; or the thinning circuit 50 and the thinning circuit 50a may be used alternately.

The operations and effects of the image storage/transmission circuit 48 will now be described further.

FIG. 7 is a schematic view for illustrating the memory addresses of the information stored in the image memory 49.

Although several thousand×several thousand pixels are provided actually, FIG. 7 has 8×8 pixels for the description.

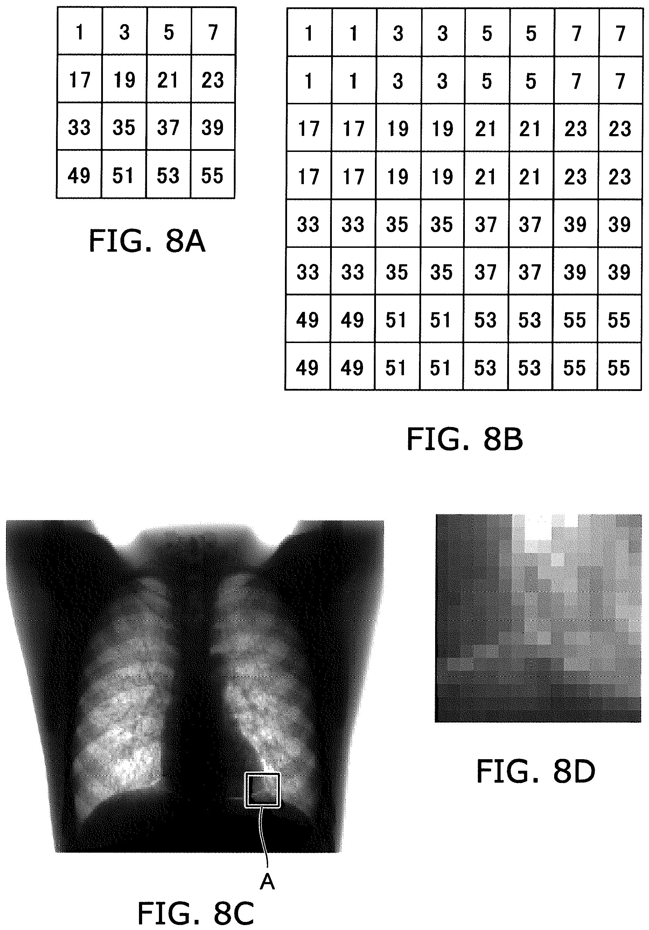

FIG. 8A to FIG. 11D are X-ray images and schematic views for illustrating the information thinned by the image storage/transmission circuit 48.

FIG. 8A to FIG. 11A are schematic views for illustrating the designated memory addresses.

FIG. 8B to FIG. 11B are schematic views for illustrating the information included in the X-ray image displayed in the display part 103. The numerical values in the figures are memory addresses.

FIG. 8C to FIG. 11C are the X-ray images displayed in the display part 103.

FIG. 8D to FIG. 11D are enlarged images of portion A of FIG. 8C to FIG. 11C.

First, the information (the information from all of the pixels 2b provided in the array substrate 2) that is read by the read circuit 43 is stored in the image memory 49.

For example, as shown in FIG. 7, the information that is stored is arranged in order from the upper left.

Then, the information stored in the image memory 49 is output by being thinned every other column and row. Therefore, the thinning circuit 50 designates the memory addresses of the image memory 49 every other column and row. The image memory 49 inputs, to the transmission output circuit 51, the information stored at the designated memory addresses. The information that is output from the image memory 49 is as shown in FIG. 8A.

Continuing, the transmission output circuit 51 transmits the information shown in FIG. 8A to the display part 103 as an X-ray image signal.

Because the information stored in the image memory 49 is transmitted by being thinned every other column and row in the embodiment, the amount of information that is transmitted is reduced to 1/4; and the transmission time also is shortened to 1/4.

The display part 103 generates the X-ray image based on the transmitted information. At this time, by the correction circuit provided in the display part 103, the information shown in FIG. 8B is generated from the information shown in FIG. 8A; and the X-ray image is generated based on the information shown in FIG. 8B. In other words, the information that is used when configuring the X-ray image is as shown in FIG. 8B. Known technology is applicable to the correction circuit provided in the display part 103; and a detailed description is therefore omitted.

As shown in FIGS. 8C and 8D, the X-ray image that is displayed is an image having low resolution because the amount of information is reduced to 1/4.

The operator of the X-ray imaging device 100 may end the image display at this point in time if the diagnosis is possible using the X-ray image having this resolution. Therefore, it is possible to complete the diagnosis in about 1/4 of the conventional time.

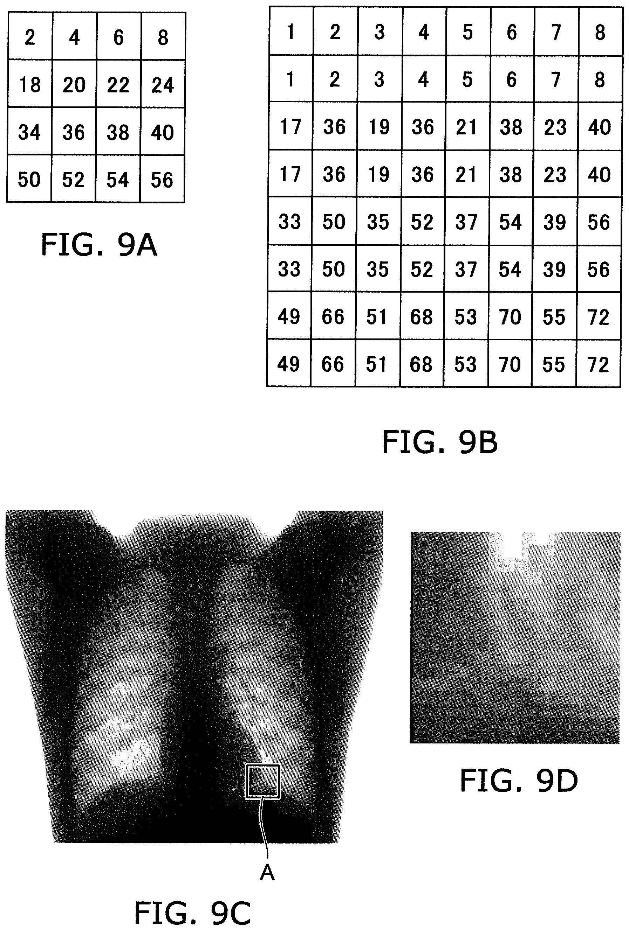

Then, in the case where the diagnosis cannot be performed using this resolution, the operator can request information to supplement the information shown in FIG. 8A. As described above, the information that was not used the previous time is stored in the image memory 49. Therefore, the information that is adjacent to the information used the previous time can be used as the supplemental information. For example, the thinning circuit 50 designates the memory addresses adjacent in the lateral direction to the memory addresses designated the previous time. The image memory 49 inputs, to the transmission output circuit 51, the information stored at the designated memory addresses. The information that is output from the image memory 49 is as shown in FIG. 9A.

Continuing, the transmission output circuit 51 transmits the information shown in FIG. 9A to the display part 103 as the supplemental information.

The display part 103 generates the X-ray image by supplementing the information shown in FIG. 8B using the information shown in FIG. 9A. In such a case, the information that is included in the X-ray image is as shown in FIG. 9B. The supplementation of the information is performed by the correction circuit provided in the display part 103.

As shown in FIGS. 9C and 9D, the resolution of the displayed X-ray image is 2 times the resolution of the X-ray image of the previous time.

If the diagnosis is possible using the X-ray image having this resolution, the operator can end the image display at this point in time.

In the case where the diagnosis cannot be performed using this resolution, the operator can further request the supplemental information.

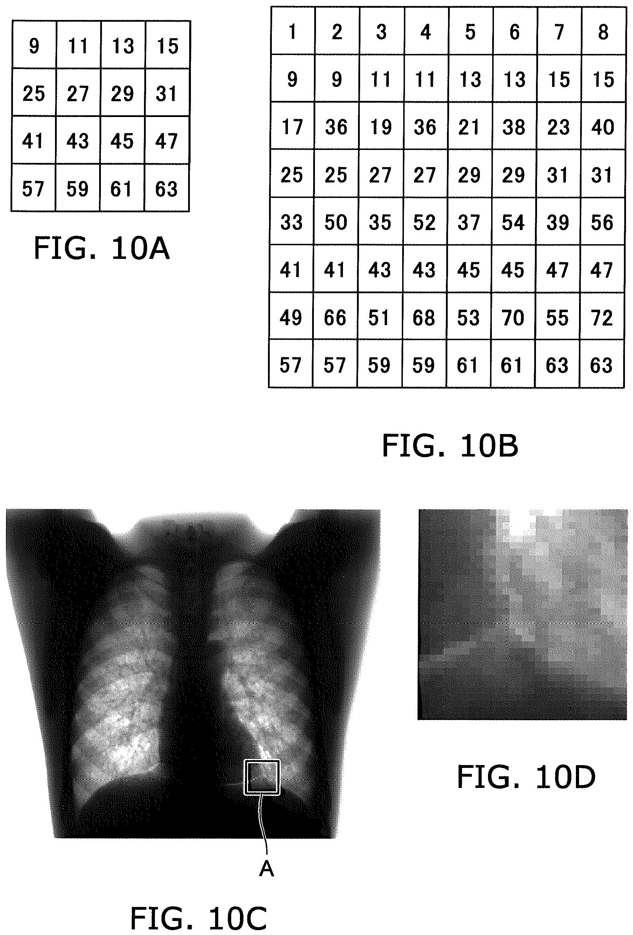

For example, the thinning circuit 50 designates the memory addresses adjacent in the longitudinal direction to the initially-designated memory addresses. The image memory 49 inputs, to the transmission output circuit 51, the information stored at the designated memory addresses. The information that is output from the image memory 49 is as shown in FIG. 10A.

Then, the transmission output circuit 51 transmits the information shown in FIG. 10A to the display part 103 as the supplemental information.

The display part 103 generates the X-ray image by supplementing the information shown in FIG. 9B using the information shown in FIG. 10A. In such a case, the information included in the X-ray image is as shown in FIG. 10B. The supplementation of the information is performed by the correction circuit provided in the display part 103.

As shown in FIGS. 10C and 10D, the resolution of the displayed X-ray image is 3 times the resolution of the initial X-ray image.

If the diagnosis is possible using the X-ray image having this resolution, the operator can end the image display at this point in time.

In the case where the diagnosis cannot be performed using this resolution, the operator can further request the supplemental information.

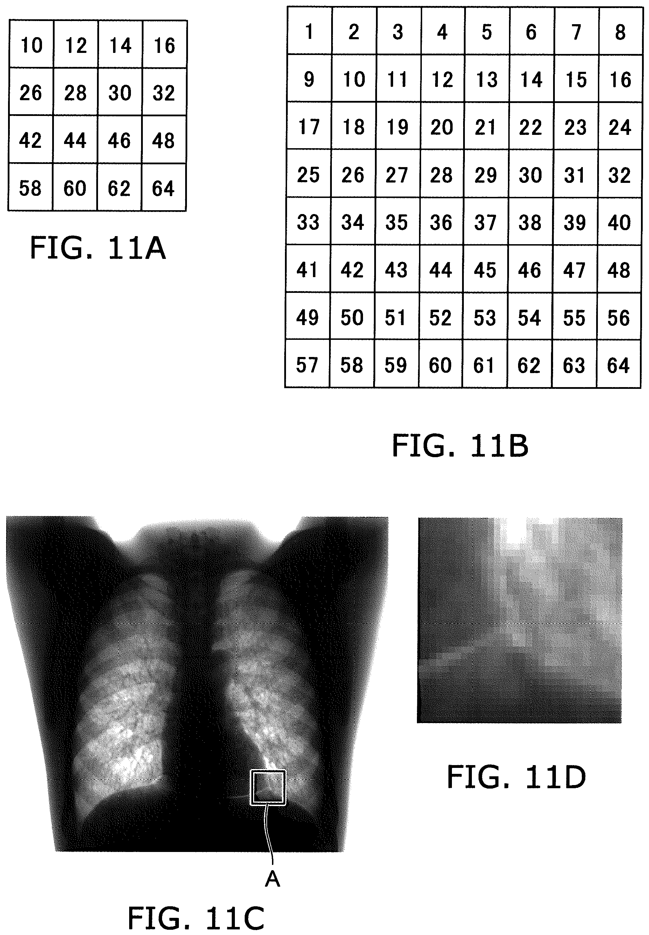

For example, the thinning circuit 50 designates the memory addresses not designated up to the previous time. The image memory 49 inputs, to the transmission output circuit 51, the information stored at the designated memory addresses. The information that is output from the image memory 49 is as shown in FIG. 11A.

Then, the transmission output circuit 51 transmits the information shown in FIG. 11A to the display part 103 as the supplemental information.

The display part 103 generates the X-ray image by supplementing the information shown in FIG. 10B using the information shown in FIG. 11A. In such a case, the information that is included in the X-ray image is as shown in FIG. 11B. The supplementation of the information is performed by the correction circuit provided in the display part 103.

As shown in FIGS. 11C and 11D, the resolution of the displayed X-ray image is 4 times the resolution of the initial X-ray image. In other words, FIGS. 11C and 11D are the X-ray image generated from all of the information stored in the image memory 49.

The request of the supplemental information may be performed by, for example, the operator inputting a signal to the thinning circuit 50 from a not-illustrated input device.

Thus, by sequentially adding the supplemental information, the resolution can be increased in stages to become the resolution of the X-ray image generated from all of the information.

Although the supplemental information is sequentially added by the determination of the operator in the case of the description recited above, the level of the supplementation may be predetermined; and the X-ray image that has the desired resolution may be displayed automatically. In such a case, it is also possible to perform a rough diagnosis using the initial X-ray image, and to perform the final diagnosis using the final X-ray image.

In the illustration recited above, the transmission time of each stage is 1/4 because the amount of information is increased 1/4 at a time. However, because several thousand×several thousand pixels are provided actually, the thinned information amount may be further increased; and the transmission time of each stage may be shortened further.

The thinned information amount also may be changed according to the memory addresses of the information stored in the image memory 49. For example, the likelihood is low that an important section is imaged in the peripheral edge region of the X-ray image; and the likelihood is high that an important section is imaged in the central region of the X-ray image. Therefore, the thinned information amount of the information stored in the image memory 49 corresponding to the peripheral edge region of the X-ray image may be high; and the thinned information amount of the information stored in the image memory 49 corresponding to the central region of the X-ray image may be low. In other words, the thinning circuit 50 may set the limit of the output of the multiple information corresponding to the peripheral edge region of the X-ray image to be larger than the limit of the output of the multiple information corresponding to the central region of the X-ray image. Thus, it is possible to perform an efficient diagnosis.

The operations and effects of the image storage/transmission circuit 48a will now be described further.

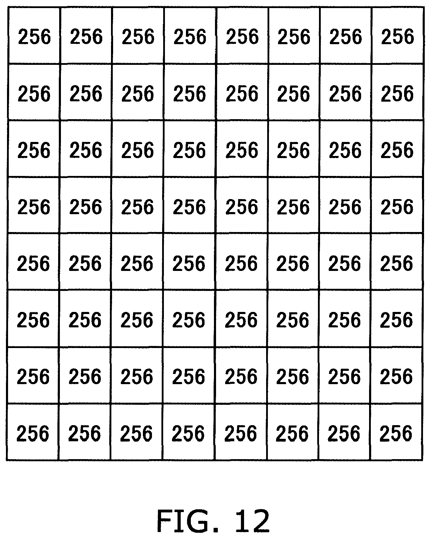

FIG. 12 is a schematic view for illustrating the gradation of the information stored in the image memory 49.

Although several thousand×several thousand pixels are provided actually, FIG. 12 has 8×8 pixels for the description. Although the gradation is actually about 65536 levels (sixteen bits), 256 levels (eight bits) are used for the description.

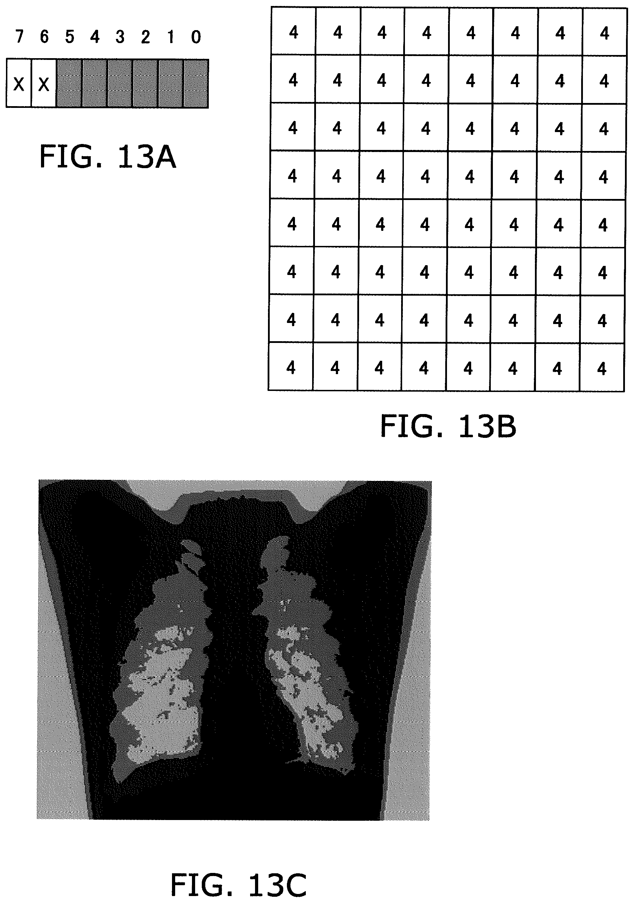

FIG. 13A to FIG. 16D are X-ray images and schematic views for illustrating the information thinned by the image storage/transmission circuit 48a.

FIG. 13A to FIG. 16A are schematic views for illustrating the designated bits. The "×" in the figures are the designated bits.

FIG. 13B to FIG. 16B are schematic views for illustrating the information included in the X-ray image displayed in the display part 103. The numerical values in the figures are the levels of the gradation.

FIG. 13C to FIG. 16C are the X-ray images displayed in the display part 103.

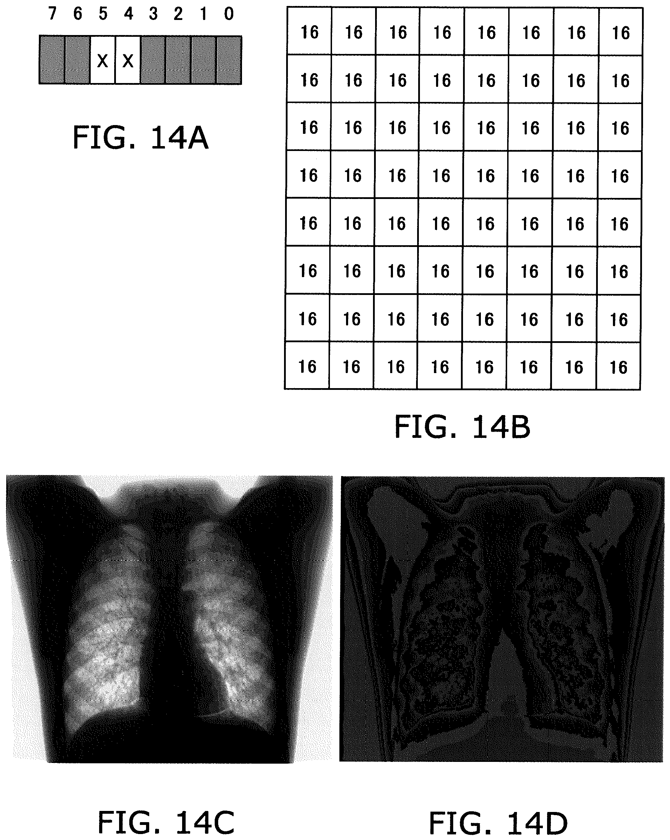

FIG. 14D to FIG. 16D are the X-ray images of the supplemental information.

First, the information (the information from all of the pixels 2b provided in the array substrate 2) that is read by the read circuit 43 is stored in the image memory 49.

For example, as shown in FIG. 12, the information that is stored is arranged in order from the upper left. The gradation of the stored information is 256 levels (eight bits).

Then, the bit numbers of the multiple information output from the image memory 49 are limited. To this end, the thinning circuit 50a limits the bit numbers of the multiple information output from the image memory 49. For example, as shown in FIG. 13A, the thinning circuit 50a inputs, to the transmission output circuit 51, the information of the gradation of the two high-order bits of the multiple information output from the image memory 49. The information that is output from the image memory 49 is as shown in FIG. 13B.

Continuing, the transmission output circuit 51 transmits the information shown in FIG. 13B to the display part 103 as the X-ray image signal.

In the embodiment, the information stored in the image memory 49 is transmitted by being thinned. In other words, two bits of the information of the gradation are transmitted from the eight bits of the information of the gradation. Therefore, the amount of information that is transmitted is reduced to 1/4; and the transmission time also is shortened to 1/4.

The display part 103 generates the X-ray image based on the information shown in FIG. 13B.

As shown in FIG. 13C, the X-ray image that is displayed is an image having a low gradation because the amount of information is reduced to 1/4.

If the diagnosis is possible using this X-ray image, the operator of the X-ray imaging device 100 can end the image display at this point in time. Therefore, it is possible to complete the diagnosis in about 1/4 of the conventional time.

In the case where the diagnosis cannot be performed using this X-ray image, the operator can request information to supplement the information shown in FIG. 13B. As described above, the information that was not used the previous time is stored in the image memory 49. Therefore, the information relating to the gradation not used the previous time can be used as the supplemental information. For example, as shown in FIG. 14A, the thinning circuit 50a inputs, to the transmission output circuit 51, two bits of the information of the gradation of lower order than that of the previous time. The information (the X-ray image of the supplemental information) that is input to the transmission output circuit 51 is as shown in FIG. 14D.

Then, the transmission output circuit 51 transmits the information shown in FIG. 14D to the display part 103 as the supplemental information.

The display part 103 generates the X-ray image by supplementing the information shown in FIG. 13B using the information shown in FIG. 14D. In such a case, the information that is included in the X-ray image is as shown in FIG. 14B.

As shown in FIGS. 14B and 14C, the levels of the gradation of the displayed X-ray image are 4 times the levels of the gradation of the X-ray image of the previous time.

If the diagnosis is possible using this X-ray image, the operator can end the image display at this point in time.

In the case where the diagnosis cannot be performed using this X-ray image, the operator can further request the supplemental information.

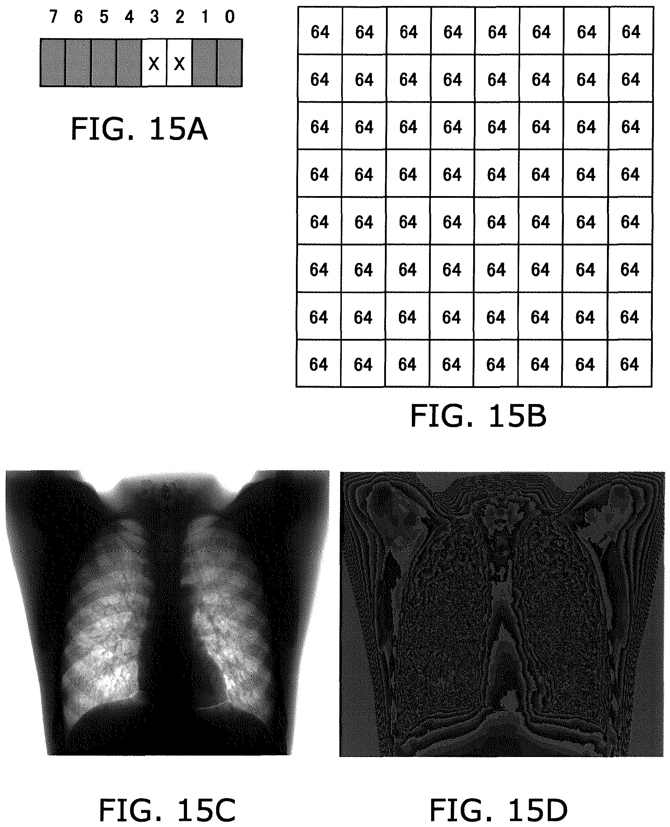

For example, as shown in FIG. 15A, the thinning circuit 50a inputs, to the transmission output circuit 51, two bits of the information of the gradation of lower order than that of the previous time. The information (the X-ray image of the supplemental information) that is input to the transmission output circuit 51 is as shown in FIG. 15D.

Then, the transmission output circuit 51 transmits the information shown in FIG. 15D to the display part 103 as the supplemental information.

The display part 103 generates the X-ray image by supplementing the information shown in FIG. 14B using the information shown in FIG. 15D. In such a case, the information that is included in the X-ray image is as shown in FIG. 15B.

As shown in FIGS. 15B and 15C, the levels of the gradation of the displayed X-ray image are 4 times the levels of the gradation of the X-ray image of the previous time.

If the diagnosis is possible using this X-ray image, the operator can end the image display at this point in time.

In the case where the diagnosis cannot be performed using this X-ray image, the operator can further request the supplemental information.

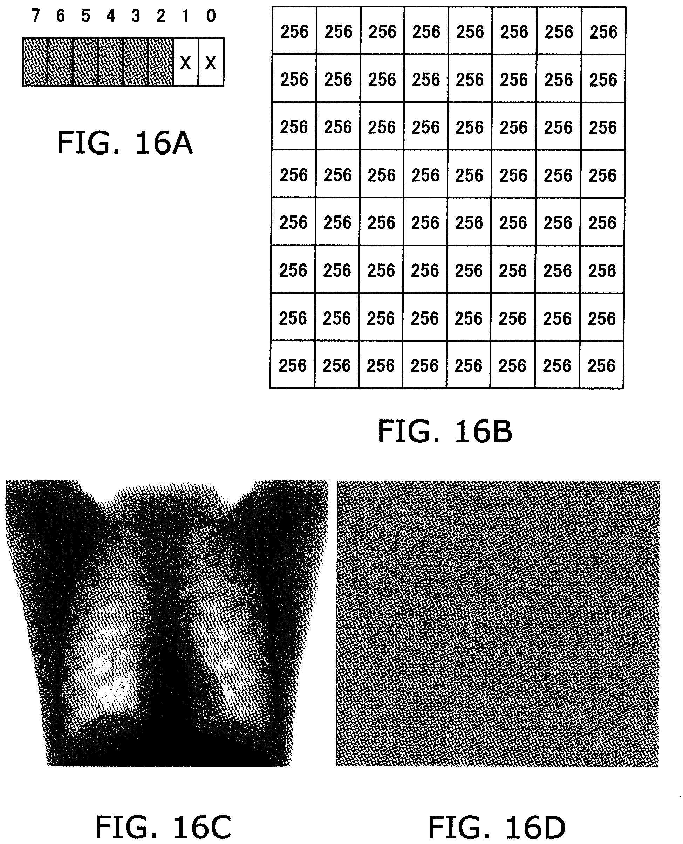

For example, as shown in FIG. 16A, the thinning circuit 50a inputs, to the transmission output circuit 51, two bits of the information of the gradation of lower order than that of the previous time. The information (the X-ray image of the supplemental information) that is input to the transmission output circuit 51 is as shown in FIG. 16D.

Then, the transmission output circuit 51 transmits the information shown in FIG. 16D to the display part 103 as the supplemental information.

The display part 103 generates the X-ray image by supplementing the information shown in FIG. 15B using the information shown in FIG. 16D. In such a case, the information that is included in the X-ray image is as shown in FIG. 16B.

As shown in FIGS. 16B and 16C, the levels of the gradation of the displayed X-ray image are 4 times the levels of the gradation of the X-ray image of the previous time. In other words, FIGS. 16B and 16C are the X-ray images generated from all of the information stored in the image memory 49.

The request of the supplemental information may be performed by, for example, the operator inputting a signal to the thinning circuit 50a from a not-illustrated input device.

Thus, by sequentially adding the supplemental information, the gradation can be increased in stages to become the gradation of the X-ray image generated from all of the information.

Although the supplemental information is sequentially added by the determination of the operator in the case of the description recited above, the level of the supplementation may be predetermined; and the X-ray image that has the desired gradation may be displayed automatically. In such a case, it is also possible to perform a rough diagnosis using the initial X-ray image, and to perform the final diagnosis using the final X-ray image.

In the illustration recited above, the transmission time of each stage is reduced to 1/4 because the gradation is increased two bits at a time (the amount of information increased 1/4 of the amount at a time). However, because several thousand×several thousand pixels are provided actually, the thinned information amount may be increased further; and the transmission time of each stage may be shortened further.

The thinned information amount also may be changed according to the memory addresses of the information stored in the image memory 49. For example, the likelihood is low that an important section is imaged in the peripheral edge region of the X-ray image; and the likelihood is high that an important section is imaged in the central region of the X-ray image. Therefore, the thinned information amount of the information stored in the image memory 49 corresponding to the peripheral edge region of the X-ray image may be high; and the thinned information amount of the information corresponding to the central region of the X-ray image may be low. In other words, the thinning circuit 50a may set the limit of the output of the multiple information corresponding to the peripheral edge region of the X-ray image to be larger than the limit of the output of the multiple information corresponding to the central region of the X-ray image. Thus, it is possible to perform an efficient diagnosis.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions. Moreover, above-mentioned embodiments can be combined mutually and can be carried out.

## Claims

1. A radiation detector (1), comprising:
a detector including a plurality of pixels (2b) and converting radiation into electrical information:
a read circuit (43) reading the information for each of the plurality of pixels (2b);
an image memory (49) storing a plurality of the read information; and
a thinning circuit (50) limiting an output of the plurality of information stored in the image memory (49).

2. The detector (1) according to claim 1, wherein the thinning circuit (50) further outputs information of the stored plurality of information not output the previous time.

3. The detector (1) according to claim 2, wherein the thinning circuit (50) outputs, in stages by dividing into multiple outputs, the plurality of information stored in the image memory.

4. The detector (1) according to any one of claims 1 to 3, wherein the thinning circuit (50) limits the output of the plurality of information according to a command or a method, the command being from an operator, the method being predetermined.

5. The detector (1) according to any one of claims 1 to 4, wherein the thinning circuit (50) controls the limiting of the output of the information according to a region of a radiation image.

6. The detector (1) according to any one of claims 1 to 5, wherein the thinning (50) circuit changes a limit amount of the output of the plurality of information based on at least one of a transmission time requested for the plurality of information, a resolution requested for a radiation image, or a gradation requested for the radiation image.

7. The detector (1) according to any one of claims 1 to 6, wherein the plurality of information stored in the image memory (49) is an image data signal of one screen of a radiation image.

8. The detector (1) according to any one of claims 1 to 7, wherein the thinning circuit (50) designates a memory address of the plurality of information to be output from the image memory (49).

9. The detector (1) according to any one of claims 1 to 8, wherein the thinning circuit (50) generates a signal designating a memory address of the plurality of information, and inputs the generated signal to the image memory (49).

10. The detector (1) according to claim 9, wherein the image memory (49) outputs the information stored at the designated memory address based on the signal designating the memory address.

11. The detector (1) according to claim 8, wherein the thinning circuit (50) outputs, in stages by dividing into multiple outputs, the plurality of information for each desired memory address.

12. The detector (1) according to any one of claims 1 to 7, wherein the thinning circuit (50) designates a bit number of the plurality of information to be output from the image memory (49).

13. The detector (1) according to any one of claims 1 to 7, wherein the thinning circuit (50) limits a bit number of the plurality of information to be output from the image memory (49).

14. The detector (1) according to claim 12, wherein the thinning circuit (50) outputs, in stages by dividing into multiple outputs, the plurality of information for each desired bit number.

15. The detector (1) according to claim 14, wherein the thinning circuit (50) outputs, in stages, from the information of a high-order bit number.

16. The detector (1) according to claim 6, wherein the thinning circuit (50) designates, in the case where the gradation of the radiation image is given priority, a memory address of the plurality of information to be output from the image memory (49).

17. The detector (1) according to claim 6, wherein the thinning circuit (50) designates, in the case where the resolution of the radiation image is given priority, a bit number of the plurality of information to be output from the image memory (49).

18. A radiographic imaging device (100), comprising:
the radiation detector (1) according to any one of claims 1 to 17;
a radiation generator (102) generating radiation and irradiating the radiation onto the detector of the radiation detector (1);
a display part (103) generating a radiation image based on a signal output from the radiation detector(1), the display part (103) displaying the radiation image that is generated.

19. The device (100) according to claim 18, wherein
the display part (103) includes a correction circuit, and
the correction circuit generates the radiation image based on a signal output from the radiation detector (1), the outputting being in stages by dividing into multiple outputs.

20. The device (100) according to claims 18 or 19, wherein the correction circuit increases, in stages and based on a signal output from the radiation detector (1), at least one of a resolution of the radiation image or a gradation of the radiation image, the signal outputting being in stages by dividing into multiple outputs.
